# EUROPEAN PATENT SPECIFICATION

(11) **EP 0 736 524 B1**
(45) Date of publication and mention of the grant of the patent: **06.11.2002**
(21) Application number: 96104685.1
(22) Date of filing: 25.03.1996
(51) Int. Cl.: C07C 309/80, C07C 303/10

(54) **Process for the synthesis of fluorinated alkyl sulphonyl halides**
Verfahren zur Herstellung von fluorierten Alkylsulfonsäurehalogeniden
Procédé pour la préparation d'halogénures d'acides alkylsulfoniques fluorés

(30) Priority: 05.04.1995 DK 38395
(43) Date of publication of application: 09.10.1996
(73) Proprietor: Haldor Topsoe A/S, 2800 Lyngby (DK)
(72) Inventor: Zavilla, John, 2980 Kokkedal (DK); Hommeltoft, Sven Ivar, 3400 Hillerod (DK)
(74) Representative: Vogelsang-Wenke, Heike, Dr.

(56) References cited:
- FR-A- 1 555 130
- FR-A- 1 573 537
- US-A- 3 542 864

## Description

The present invention is directed to the preparation of fluorinated alkyl sulphonyl halides. In particular, the invention provides a process for the preparation of those compounds by employing a specific solvent to obtain high yield in the synthesis of fluorinated alkyl sulphonyl halides.

Polyfluoro and perfluoro alkyl sulphonyl fluorides are presently prepared by a number of methods, including electrochemical fluorination of the corresponding alkyl sulphonyl halides or cyclic sulphones, using anhydrous HF (US Patent No. 2,732,398, DE Offenlegungsschrift No. 1,912,738, DE Offenlegungsschrift No. 2,725,211, DE Offenlegungsschrift No. 4,208,364, DE Offenlegungsschrift No. 4,218,562, DE Offenlegungsschrift No. 4,226,758) or elemental fluorine (Inorg. Chem. 30 (1991) 789-794).

It is further known to use a multistep synthesis, where the final step is the reaction of the corresponding perfluoro alkyl sulphonyl chloride with cesium fluoride, giving the acid fluoride (Zh. Org. Khim. 14 (1978) 275-278). Preparation of polyfluorinated alkyl sulphonyl fluorides by addition of sulphuryl chloride fluoride to a perfluoro-olefin is disclosed in US Patent No. 2,877,267 and perfluorinated alkyl sulphonyl fluorides by addition of sulphuryl fluoride to a perfluoro-olefin is disclosed in FR Patent No. 1,573,537, US Patent No. 3,542,864, DE Auslegeschrift No. 1,668,584 and J. Org. Chem. 33 (1968) 344-346.

By electrochemical fluorination, it is possible to synthesize linear perfluoro-alkyl sulphonyl fluorides of variable chain length, whereas acid fluorides having a branched perfluoroalkyl chain only are obtainable in poor yields. Thus, it is reported that perfluoro-i-propyl sulphonyl fluoride has been prepared by the direct fluorination of i-propyl sulphonyl fluoride using elemental fluorine (Inorg. Chem. 30 (1991) 789-794).

The reaction is carried out in a four-zone cryogenic reactor with a reaction time of 9 days, at temperatures between ö100°C and ambient, yielding 0,96 g (29%) of perfluoro-i-propyl sulphonyl fluoride.

A further synthesis route for the preparation of branched perfluoro alkyl sulphonyl fluorides comprises addition of sulphuryl fluoride to perfluoro-olefins in a suitable solvent in the presence of catalytic amounts of fluoride ions, e.g. synthesis of perfluoro-i-propyl sulphonyl fluoride by the addition of sulphuryl fluoride to perfluoropropene using different combinations of solvent and fluoride donors.

We have now found that fluorinated alkyl sulphonyl halides are prepared with a considerably higher yield than by the known processes, when carrying out the reaction of sulphuryl halide with fluorinated unsaturated hydrocarbons in the presence of catalytic amounts of fluoride salts and in a solvent comprising alkyl sulphonyl and/or alkyl sulphoxide compounds.

Pursuant to this finding, the invention provides an improved process for the preparation of fluorinated alkane sulphonyl halides having the general formula: 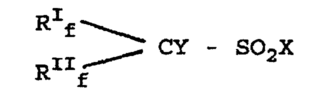 where R^{I}_{f} is a fluorinated alkyl group, R^{II}_{f} is a fluorinated alkyl group or fluorine, X is a halogen atom, and Y is a proton, a halogen atom, or a fluorinated alkyl group,
by reacting a corresponding fluorinated unsaturated hydrocarbon with a sulphuryl halide, wherein the reaction is carried out in the presence of at least catalytic amounts of fluoride in a solvent comprising alkyl sulphonyl and/or alkyl sulphoxide compounds.

Solvents used in the known synthesis processes are diethyleneglycol dimethylether, acetonitrile and N,N-dimethylformamide, which belong to the category of aprotic polar solvents. Those solvents have a moderately high dielectric constant and the ability to dissolve at least catalytic amounts of inorganic salts.

The alkyl sulphonyl and/or alkyl sulphoxide compounds, used in the invention, have a similar dielectric constant to that of the solvents used in the known processes.

As a theoretical explanation, the higher product yield obtained by use of alkyl sulphonyl and/or alkyl sulphoxide compounds as solvent in the synthesis of fluorinated alkane sulphonyl halides may be provided through the presence of -SO- or -SO₂- groups or more specific through the structure S=O, which by its configuration is similar to the structure of the sulphuryl halide reagent and the fluorinated alkyl sulphonyl halide product.

Presently, the most preferred compound is tetramethylene sulphone as solvent for use in the above process.

### Example 1:

### Preparation of perfluoro-i-propyl sulphonyl fluoride Chemicals

Perfluoropropene (-29°C), Heraeus. Sulphuryl fluoride (bp: -53°C), synthesized in the lab. Tetramethylene sulphone (99%, mp: 28°C, bp: 285°C), Heraeus. Dried with molecular sieves (3Å). Potassium fluoride, Riedel-de Haën. Dried at 200°C (48 hr).

Into a clean dry Parr-reactor (stainless steel, vol: 2 litres) 400 ml of tetramethylene sulphone were loaded together with 46 g of potassium fluoride. The reactor was sealed, flushed with dry nitrogen, evacuated, cooled in a dry ice/acetone mixture and loaded with 565 g of sulphuryl fluoride. The reactor was heated to 30°C, placed in an oven and heated to 140°C under vigorous stirring. 635 g of perfluoropropene were then introduced into the reactor over a period of 6 hr, resulting in a pressure drop from 995 psi to 508 psi. Subsequently, the reaction mixture was cooled and distilled at atmospheric pressure. A product fraction was recovered within the boiling range of 0-39°C as 1032,2 g colourless liquid consisting of (CF₃)₂CFSO₂F including small amounts of (CF₃)₂CFH. Product yield : 72%.

## Claims

1. Process for the preparation of fluorinated alkyl sulphonyl halides having the general formula: 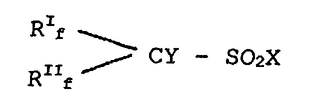 where R^{I}_{f} is a fluorinated alkyl group and R^{II}_{f} is a fluorinated alkyl group or fluorine, X is a halogen atom, and Y is a proton, a halogen atom, or a fluorinated alkyl group,
by reaction of a corresponding fluorinated unsaturated hydrocarbon with sulphuryl halide, wherein the reaction is carried out in the presence of at least catalytic amounts of a fluoride in a solvent comprising alkyl sulphonyl and/or alkyl sulphoxide compounds.

2. Process according to claim 1, wherein the solvent comprises tetramethylene sulphone.

3. Process according to claim 1, wherein the sulphuryl halide is a sulphuryl fluoride halide.

4. Process according to claim 1, wherein the sulphuryl halide is sulphuryl fluoride.

5. Process according to claim 1, wherein the fluoride is an alkali metal fluoride.

6. Process according to claim 1, wherein the fluoride is potassium fluoride.

## Patentansprüche

1. Verfahren zur Herstellung von fluorierten Alkylsulfonylhalogeniden der allgemeinen Formel 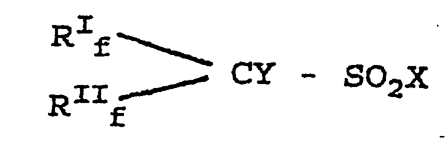 worin R^{I}_{f} eine fluorierte Alkylgruppe und R^{II}_{f} eine fluorierte Alkylgruppe oder Fluor ist, X ein Halogenatom ist, und Y ein Proton, ein Halogenatom oder eine fluorierte Alkylgruppe ist, durch Umsetzen eines entsprechend fluorierten, ungesättigten Kohlenwasserstoffs mit Sulfurylhalogenid, worin die Reaktion in Gegenwart von wenigstens katalytischen Mengen eines Fluorids in einem Lösungsmittel durchgeführt wird, das Alkylsulfonyl- und/oder Alkylsulfoxidverbindungen enthält.

2. Verfahren nach Anspruch 1, worin das Lösungsmittel Tetramethylensulfon enthält.

3. Verfahren nach Anspruch 1, worin das Sulfurylhalogenid ein Sulfurylfluoridhalogenid ist.

4. Verfahren nach Anspruch 1, worin das Sulfurylhalogenid Sulfurylfluorid ist.

5. Verfahren nach Anspruch 1, worin das Fluorid ein Alkalimetallfluorid ist.

6. Verfahren nach Anspruch 1, worin das Fluorid Kaliumfluorid ist.

## Revendications

1. Procédé pour la préparation d'halogénures d'alkyl sulfonyle fluorés ayant la formule générale : 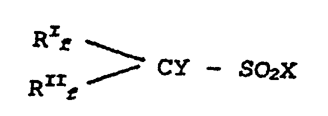 dans laquelle R^{I}_{f} est un groupe alkyle fluoré, R^{II}_{f} est un groupe alkyle fluoré ou le fluor, X est un atome d'halogène, et Y est un proton, un atome d'halogène ou un groupe alkyle fluoré,
par réaction d'un hydrocarbure insaturé fluoré correspondant avec un halogénure de sulfuryle,
dans lequel la réaction est effectuée en présence de quantités au moins catalytiques d'un fluorure dans un solvant comprenant des composés alkyl sulfonyles et/ou alkyl sulfoxydes.

2. Procédé selon la revendication 1, dans lequel le solvant comprend de la tétraméthylène sulfone.

3. Procédé selon la revendication 1, dans lequel l'halogénure de sulfuryle est un halogénure fluorure de sulfuryle.

4. Procédé selon la revendication 1, dans lequel l'halogénure de sulfuryle est le fluorure de sulfuryle.

5. Procédé selon la revendication 1, dans lequel le fluorure est un fluorure de métal alcalin.

6. Procédé selon la revendication 1, dans lequel le fluorure est le fluorure de potassium.
